# EUROPEAN PATENT SPECIFICATION

(11) **EP 2 916 766 B1**
(45) Date of publication and mention of the grant of the patent: **12.10.2016**
(21) Application number: 13798373.0
(22) Date of filing: 06.11.2013
(51) Int. Cl.: A61B 50/30

(54) **STORAGE DEVICE**
SPEICHERVORRICHTUNG
DISPOSITIF DE RANGEMENT

(30) Priority: 09.11.2012 GB 201220206
(43) Date of publication of application: 16.09.2015
(73) Proprietor: The Ruhof Corporation, Mineola NY 11501 (US)
(72) Inventor: RAMSEY, Peter, Chesterfield Derbyshire S40 1QX (GB)
(74) Representative: Dummett Copp LLP
(86) International application number: PCT/GB2013/052907
(87) International publication number: WO 2014/072706

(56) References cited:
- EP-A2- 0 317 047
- WO-A1-02/091937
- WO-A1-2007/077172
- WO-A1-2010/128554
- WO-A1-2012/126973
- US-A1- 2011 073 507

## Description

### BACKGROUND

### a. Field of the Invention

The present invention relates to a kit for the storage and transportation of medical equipment. In particular, the present invention relates to a single-use storage device for endoscopes.

### b. Related Art

Flexible medical endoscopes are used for the internal examination of various parts of the human or animal body. They are produced in diameters ranging from 0.02 to 0.6 inches (0.5 to 15 mm) and with lengths of 12 to 120 inches (300 to 3000 mm). The majority of endoscopes have internal channels, down which air, water or accessories may be directed so as to facilitate examinations, or to carry out surgical procedures.

Due to the invasive nature of many of the procedures for which flexible medical endoscopes are used, it is necessary that the endoscopes and all the detachable parts and components such as the valves are thoroughly cleaned and disinfected prior to and after each use. It is desirable if the room in which the cleaning and disinfection are carried out is in close proximity to the operating theatre or procedure room; however, this is often not the situation and as a result, endoscopes are frequently carried over reasonably long distances both prior to and after being used on a patient.

At least in the United Kingdom and France, the recent BSE (Bovine Spongiform Encephalopathy) crisis has led to heightened concerns that the human form, Creutzfeldt-Jakob Disease (CJD), may be transmitted by contaminated endoscopes or their detachable parts. Moreover, the recent re-emergence of tuberculosis also presents a threat of airborne contamination in areas where endoscopes are being used and transported.

A number of pieces of apparatus are known to reduce contamination of medical equipment or to aid in the disposal of medical equipement after use. WO 02/091937 discloses packaging for disposable medical equipment; EP 0317047 discloses a waste bag which may be used for clinical waste; US 2011/0073507 discloses a protective sterile drape for a surgical tray stand. The two-part form of independent claims 1, 8 and 15 is based on this document; and WO 2010/128554 discloses a container for medical instruments.

Several national and international clinical guidelines regarding the use, storage and cleaning of endoscopes have recently been published. These include:
- National Endoscopy Programme Decontamination Standards for Flexible Endoscopes, updated March 2009, L. Thomson *et al.*
- Multisociety Guideline on Reprocessing Flexible GI Endoscopes, 2011, Bret T. Petersen *et al.*
- ESGE ESGENA guideline, Cleaning and disinfection in gastrointestinal endoscopy, update 2008, U. Beilenhoff *et al.*
- Department of health Choice Framework for local Policy and Procedures 01-06 - Decontamination of flexible endoscopes: Operational management manual 13536: 1.0: England.

Many of the current methods of carrying endoscopes are unsatisfactory for a number of reasons including:
- limited protection of the endoscope against accidental damage or contamination;
- limited protection for users against contamination and possible infection from a used endoscope; and
- limited protection for clean endoscopes against cross-contamination from used endoscopes or other potentially contaminated surfaces.

Furthermore, to reduce the possibility of cross-contamination and to allow accurate records to be kept regarding how and when the endoscope has been used, it is necessary to keep full traceability records.

It is an object of the present invention, therefore, to provide an improved means and method of storing and transporting medical equipment such as endoscopes.

### SUMMARY OF THE INVENTION

According to the invention there is provided a method of storing medical equipment using a kit, as defined in appended claim 1, comprising a bag made from a flexible plastics material, a first closure device for securing the bag closed and a second closure device for securing the bag closed, the method comprising:
a) placing an item of medical equipment in a first, clean state within the bag;
b) securing the bag closed with the first closure device;
c) removing the first closure device;
d) removing the medical equipment from the bag for use;
e) placing said used medical equipment, in a second, dirty state, in the bag; and
f) securing the bag closed with the second closure device, such that the bag is sealed to retain moisture within the bag,
wherein the first closure device comprises a distinguishing means and an identification means, the second closure device comprises a distinguishing means and an identification means, the distinguishing means of the first and second closure devices being different.

Preferably the first and second closure devices are single use and the step of removing the first closure device comprises breaking the first closure device.

The first closure device comprises a distinguishing means and an identification means, and the second closure device comprises a distinguishing means and an identification means, the distinguishing means of the first and second closure devices being different. The identification means is used to identify and trace the item of medical equipment and, as such, the method preferably further comprises associating the identification means of the first and second closure devices with the item of medical equipment to enable identification of said medical equipment when it is sealed within the bag.

In some embodiments the kit further comprises a sheet of absorbent material, and the method preferably comprises placing the sheet of absorbent material into the bag before placing the item of medical equipment in the bag. The absorbent material can, therefore, absorb any liquid on the surface of the piece of medical equipment. Typically the sheet of absorbent material will be placed into the bag before the item of used medical equipment is placed in the bag

Preferably the closure devices are cable ties. In embodiments in which the closure devices are cable ties and the cable ties each include a tab portion, the step of removing the first closure device preferably comprises pulling the tab portion so as to break the cable tie. This enables the closure device to be removed from the bag without needing to cut the closure device.

Typically the item of medical equipment is an endoscope.

Also according to the present invention there is provided a kit for the storage of medical equipment, as defined in appended claim 8, the kit comprising:
- a bag made from a flexible plastics material;
- a first closure device for securing the bag closed, the closure device being single use and the closure device having a distinguishing means and an identification means; and
- a second closure device for securing the bag closed, the closure device being single use and the closure device having a distinguishing means and an identification means,
wherein, the distinguishing means of the first and second closure devices are different.

Typically a single piece of medical equipment or a piece of medical equipment together with its accessories will be stored and transported within the bag. The closure devices are used to seal the bag closed and the distinguishing means are used to indicate whether the contents of the bag are clean or dirty. The distinguishing means may comprise lettering on a part of each of the first and second closure devices.

Preferably the identification means of the first and second closure devices are interrelated. More preferably, and in order to uniquely identify the piece of medical equipment stored within the bag, the identification means of the first and second closure devices are identical to enable traceability of the medical equipment during use.

For ease of use and cost effectiveness the first and second closure devices are preferably cable ties. However, to prevent the need to cut the cable ties to remove them from the bag, the cable ties preferably each include a tab portion, the tab portion being arranged such that, in use, a force applied to the tab portion breaks the cable tie.

It is advantageous if the bag has a rectangular base to allow the medical equipment to be more easily placed within the bag while the bag is supported on an appropriate surface. Additionally, the kit may further comprise a sheet of absorbent material, which can be laid in the base of the bag prior to the medical equipment.

In preferred embodiments, all of part of the kit is sterilised before use.

The invention also provides an assembly, as defined in appended claim 15, comprising:
- a bag made from a flexible plastics material;
- first and second closure devices for securing the bag closed, the closure devices each being single use and the closure devices each having a distinguishing means and an identification means, the distinguishing means of the first and second closure devices being different; and
- a piece of medical equipment contained within the bag;
wherein, one of the first and second closure devices is secured around the bag to seal the bag closed.

The assembly is primarily designed for applications in which the piece of medical equipment comprises an endoscope; however, the piece of medical equipment may be any suitable medical equipment they may be contained within the bag.

Preferably the identification means of the first and second closure devices are interrelated. More preferably, and in order to uniquely identify the piece of medical equipment stored within the bag, the identification means of the first and second closure devices are identical to enable traceability of the medical equipment during use. The distinguishing means are typically used to indicate whether the contents of the bag are clean or dirty, and the distinguishing means preferably comprises lettering on a part of each of the first and second closure devices.

For ease of use the first and second closure devices are preferably cable ties. However, to prevent the need to cut the cable ties to remove them from the bag, the cable ties preferably each include a tab portion, the tab portion being arranged such that, in use, a force applied to the tab portion breaks the cable tie.

It is advantageous if the bag has a rectangular base. Additionally the assembly may further comprise a sheet of absorbent material positioned in the bag for absorbing moisture from the piece of medical equipment.

### BRIEF DESCRIPTION OF THE DRAWINGS

The invention will now be further described, by way of example only, and with reference to the accompanying drawings, in which:
Figure 1 shows a storage kit for medical equipment according to an embodiment of the present invention; the kit being in use with an endoscope and a first closure device placed within a storage bag;
Figure 2 shows the storage bag of Figure 1 secured closed with a second closure device;
Figure 3 shows a pair of closure devices according to an embodiment of the present invention; and
Figure 4 shows a part of the two closure devices of Figure 3, with the closure devices separated for use.

### DETAILED DESCRIPTION

The kit 10 and method of the present invention is designed to provide an easy and cost effective storage means for enclosing and transporting medical equipment, such as endoscopes.

The kit 10 comprises a storage bag 12 that is made from a flexible plastics material. The material of the bag 12 preferably has sufficient barrier properties so as to be able to retain moisture within the bag 12 when the bag is closed. The plastics material is preferably food-grade plastic. Additionally, the bag 12 is resistant to cleaning and sterilisation fluids to which it may be exposed during cleaning and processing of the medical equipment. In this example, the plastics material is transparent so that the contents of the bag 12 can be seen at all times; however, in other embodiments the bag 12 may be printed with graphics or text, which may include warning symbols and/or instructions for use.

The bag 12 has a rectangular or square base 14, facilitating placement of the bag 12 on a supporting surface and facilitating the placement of medical equipment, for example an endoscope 16, into the bottom of the bag 12. In this example, the bag base 14 has dimensions of about 100 cm x 50 cm; however, in other embodiments the bag 12 may be of any suitable size.

The kit 10 further includes two closure devices 18, 20, which are each single use. The closure devices 18, 20 are used to seal the bag 12 closed and permit the identification of the medical equipment held within the bag 12.

In this example, the closure devices 18, 20 are cable ties 18, 20 having a head portion 22, 22' and a tail portion 24, 24'. The tail portion 24, 24' includes engagement means 26 which, in use, engage with corresponding engagement means 28 in the head portion 22, 22'. The engagement means 26, 28 are arranged such that the tail portion 24, 24' can be engaged with the head portion 22, 22' so as to form a closed loop in the cable tie 18, 20, but the tail portion 24, 24' cannot be disengaged from the head portion 22, 22' without permanently disabling or breaking the cable tie 18, 20, as described further below. In this way, the engagement means 26, 28 forms a one-way locking means and the closure devices 18, 20 are single use.

In this embodiment, the engagement means 26 of the tail portion 24, 24' is in the form of a series of protrusions or barbs and the engagement means 28 of the head portion 22, 22' is in the form of a slot including a detent feature. The protrusions on the tail portion 24, 24' are shaped such that they can be pushed through the slot in a first direction but cannot be withdrawn from the slot in an opposing direction, due to the engagement of the protrusions with the detent.

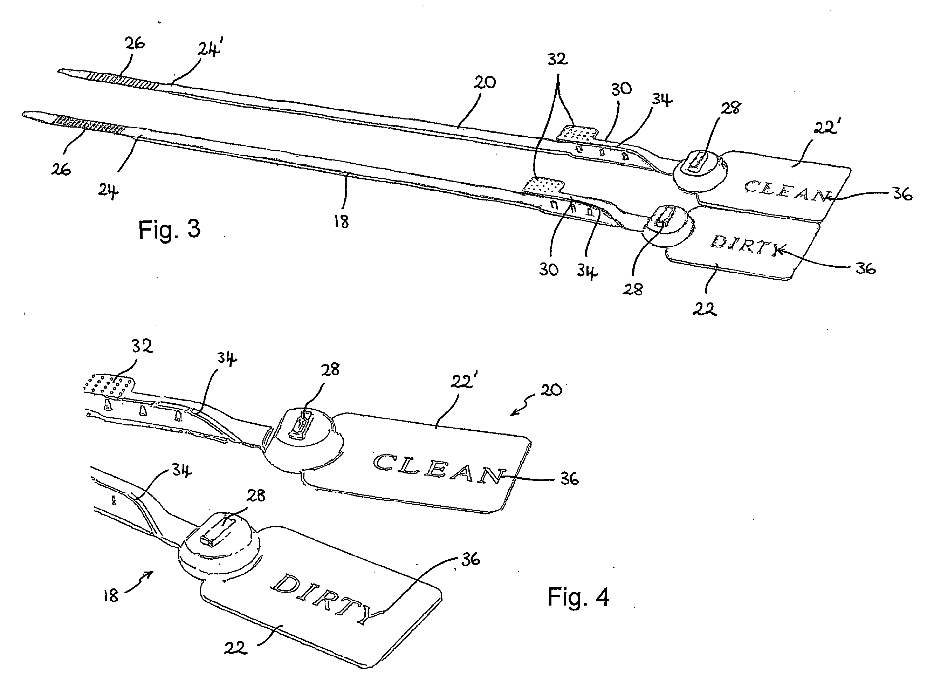

Figures 3 and 4 illustrate one embodiment of a closure device 18, 20 having a 'break to open' feature. Each cable tie 18, 20 includes a tab portion 30 that is located between the head portion 22, 22' and the tail portion 24, 24' of the cable tie 18, 20. The tab portion 30 includes a grip portion 32 suitable for gripping between a thumb and finger of a user of the kit 10. The tab portion 30 further includes a line of weakness 34 in the form of a thinner section of plastics material that spans the width of the cable tie 18, 20. The tab portion 30 is designed such that, as a user pulls the grip portion 32, the cable tie 18, 20 tears along the line of weakness 34 thereby breaking the cable tie 18, 20 such that the cable tie 18, 20 cannot be used again. In some embodiments the line of weakness 34 may be provided by a line of perforations.

Each of the cable ties 18, 20 also includes distinguishing means 36 to enable the cable ties 18, 20 to be distinguished from each other. In some embodiments the distinguishing means 36 are in the form of colours, with one of the cable ties 18 being red and the other cable tie 20 being green. In other embodiments, the distinguishing means 36 may additionally or alternatively include symbols, lettering or numbers to enable the two cable ties 18, 20, or other closure devices, to be distinguished. For example, the head portion 22' of one of the cable ties 20 may include the word CLEAN and the head portion 22 of the other cable tie 18 may include the word DIRTY. This is illustrated in the embodiment shown in Figures 3 and 4.

Furthermore, each of the cable ties 18, 20 includes associated or interrelated identification means (not shown). This identification means allows identification and traceability of the medical equipment held within the bag 12. The identification means may comprise a unique serial number, a barcode or other suitable means to identify the specific piece of medical equipment. The identification means on each of the two cable ties 18, 20 may be identical, or the identification means may be interrelated, for example including consecutive or related serial numbers (e.g. 123456A and 123456B). The use of interrelated identification means permits the piece of medical equipment to be identified and the state of the equipment to be determined, e.g. clean or dirty. The cable ties 18, 20 may, optionally, include means for identifying or recording a time at which the medical equipment is placed into the bag 12.

The ability to uniquely identify the medical equipment held within the bag 12 and to determine the state of the equipment is particularly important for endoscopes, as it is a requirement to record each step of the cleaning and decontamination cycle of the endoscope.

In this example, before use, the two closure devices 18, 20 are joined together so as to form a unique pair. The two closure devices 18, 20 are preferably joined along only a part of their length, such that the closure devices 18, 20 are easily separated. Figure 3 shows two such closure devices 18, 20 in the form of cable ties 18, 20, as previously described. In this example the head portions 22, 22' of the cable ties 18, 20 are joined along their length by a weakened and thinner section 38 of plastics material. This weakened joint 38 between the two cable ties 18, 20 may be easily broken by hand by a user of the kit 10 so that the two cable ties 18, 20 are separated for use. In other embodiments, the weakened joint may be formed by a line of perforations between the two cable ties 18, 20. The advantage of the two closure devices 18, 20 being joined in this way before use is that they remain as a unique pair. This enables suitable identification means, as described above, to be pre-applied to the closure devices. In other embodiments the two closure devices 18, 20 may be provided separately before use.

The use of the kit 10 will now be described in relation to the storage and transportation of an endoscope 16.

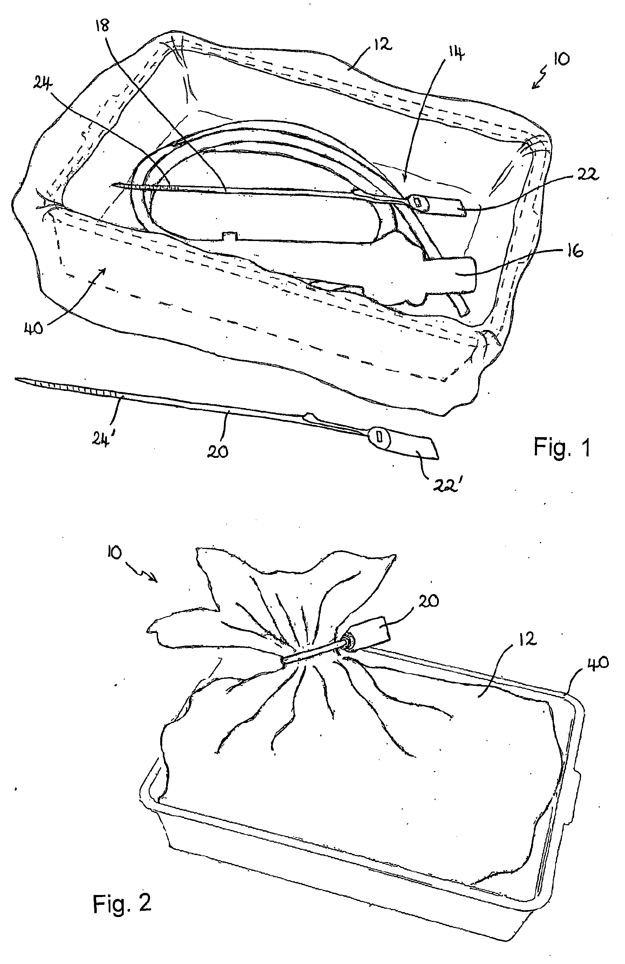

The kit 10 is designed to initially be used to store a cleaned endoscope 16 ready for use. The storage bag 12 is opened such that the endoscope 16 may be placed inside without coming into contact with the outside surface of the bag 12. In a preferred embodiment, the base 14 of the bag 12 is placed within a suitably sized tray 40 and the sides of the bag 12 are folded or rolled down so that they cover the sides of the tray 40. The endoscope 16 is then laid within the bag 12 in the base of the tray 40. This is illustrated in Figure 1. Optionally, a sheet or pad of absorbent material may be placed within the bag 12 prior to the endoscope 16. This absorbent layer acts to absorb any moisture from the endoscope 16 and also provides a layer of protection while the endoscope 16 is stored within the bag 12.

The pair of closure devices 18, 20 is separated, and a first one 18 of the two closure devices 18, 20 is placed within the bag 12 together with the clean endoscope 16. The closure device 18 placed inside the bag 18 is the one used to indicate that the endoscope 16 is used and dirty and, therefore, in this example is the red coloured cable tie 18.

The sides of the bag 12 are then brought together and the bag 12 is sealed closed with the second closure device 20. This closure device 20 indicates that the endoscope 16 within the bag 12 is clean and, in this example, is the green coloured cable tie 20. The sealed bag 12 is illustrated in Figure 2.

The endoscope 16 may then be transported fully enclosed within the bag 12 to wherever it is needed. If desired, the bag 12 may be carried within the tray 40. In a further embodiment of the invention, an outer bag or pouch (not shown) is provided. This outer pouch includes handles and is sized to receive the storage bag 12 within it. The outer pouch enables the storage bag 12 to be carried easily and also provides additional support and protection which may be desirable, in particular, for larger or heavier pieces of medical equipment.

In order to remove the endoscope 16 from the bag 12, a user must break the cable tie 20 around the bag 12. Because the break in the cable tie 20 is permanent, the cable tie 20 cannot be reused and must be disposed of. This reduces the likelihood of cross-contamination of the endoscope 16 through opening and reclosing of the bag 12 prior to use.

After it has been used, the endoscope 16 is placed back inside the bag 12. The first closure device 18 is then sealed around the bag 12. This closure device 18 indicates that the endoscope 16 is used and dirty. The endoscope 16, enclosed within the bag 12, may then be transported to suitable cleaning facilities.

When sealing the bag 12 closed, the closure device 18, 20 should be secured as tightly as possible around the bag 12. This minimises any likelihood of contamination because the endoscope 16 is fully enclosed within the bag 12. Additionally, when a used endoscope 16 is contained within the bag 12 it is preferable if the bag 12 is sealed sufficiently so that the endoscope 16 is kept moist. The moisture that is retained within the bag 12 facilitates the subsequent cleaning of the endoscope 16.

As before, the cable tie 18 must be broken to remove it from the bag 12 to enable the subsequent cleaning and disinfection of the endoscope 16. Once the endoscope 16 has been cleaned, a new kit 10 is used to store the endoscope 16 ready for use.

In some situations it may be unnecessary or undesirable to place an absorbent pad or sheet of absorbent material within the bag 12 when the endoscope 16 is clean. As such, a sheet of absorbent material may only be positioned within the bag before a dirty endoscope 16 is placed in the bag 12.

It will also be appreciated that in some situations it is preferable not to place the first closure device 18 in the bag 12 with the clean endoscope 16 to minimise any possibility of contamination. In these cases, the first closure device 18 may be retained with the bag 12 either by being placed in the tray 40 or in the outer pouch, if provided.

Although in the preceding embodiments the closure devices 18, 20 have comprised cable ties, the closure devices may be of any suitable type and may include, for example, a cable lock, single use padlock or an elasticated band.

In some embodiments it may be desirable if the kit includes a third closure device (not shown). This third closure device also includes distinguishing means, to enable the closure device to be distinguished from the first and second closure devices 18, 20, and identification means at least similar to those of the first and second closure devices 18, 20. In particular, the identification means of the third closure device may be related to or identical to the identification means of the first and second closure devices. The set of three closure devices may be used to distinguish whether the endoscope, or other piece of medical equipment, within the bag is used and dirty, clean and wet, or clean and dry. The three closure devices may be coloured red, blue and green for example.

The present invention has been developed particularly for use with endoscopes and hence is described herein with particular reference to that use, it will be appreciated, however, that the present invention may also be used for the safe storage and transportation of other medical equipment.

Providing interrelated identification means on each of the closure devices means that the medical equipment, in this case the endoscope, may be traced easily throughout its use, allowing the required records to be efficiently maintained within a hospital or other healthcare facility. It will be appreciated that in some cases it is not necessary to provide interrelated or identical identification means on each of the closure devices because, for example, the actual identification means (e.g. serial number) can simply be recorded at each stage in the process in relation to that specific piece of medical equipment.

The kit and method of the present invention, therefore, overcomes many of the problems associated with the current storage and transportation of medical equipment. In particular, the present invention provides:
i) a bag for enclosing the medical equipment to prevent contamination of the equipment when in a clean state and to prevent contamination of external surfaces when the equipment is in a dirty state,
ii) single use closure devices to prevent unnecessary and unauthorised opening of the bag, and
iii) means to uniquely identify the medical equipment held within the storage bag for traceability.

## Claims

1. A method of storing medical equipment using a kit (10) comprising a bag (12) made from a flexible plastics material, a first closure device (18) for securing the bag closed and a second closure device (20) for securing the bag closed, the method comprising:
a) placing an item of medical equipment in a first, clean state within the bag (12);
b) securing the bag (12) closed with the first closure device (18);
c) removing the first closure device (18);
d) removing the medical equipment from the bag (12) for use;
e) placing said used medical equipment, in a second, dirty state, in the bag (12); and
f) securing the bag (12) closed with the second closure device (20), such that the bag is sealed to retain moisture within the bag (12),
**characterized in that** the first closure device (18) comprises a distinguishing means(36) and an identification means, the second closure device (20) comprises a distinguishing means (36) and an identification means, the distinguishing means (36) of the first and second closure devices (18, 20) being different.

2. A method as claimed in Claim 1, wherein the first and second closure devices (18, 20) are single use and the step of removing the first closure device (18) comprises breaking the first closure device (18).

3. A method as claimed in Claim 1 or Claim 2, wherein the method further comprises associating the identification means of the first and second closure devices (18, 20) with the item of medical equipment to enable identification of said medical equipment when it is sealed within the bag (12).

4. A method as claimed in any preceding claim, wherein the kit (10) further comprises a sheet of absorbent material and wherein the method comprises placing the sheet of absorbent material into the bag (12) before placing the item of used medical equipment in the bag (12).

5. A method as claimed in any preceding claim, wherein the closure devices (18, 20) are cable ties.

6. A method as claimed in Claim 5 wherein the cable ties (18, 20) each include a tab portion (30) and wherein the step of removing the first closure device (18) comprises pulling the tab portion (30) so as to break the cable tie (18).

7. A method as claimed in any preceding claim, wherein the item of medical equipment is an endoscope (16).

8. A kit (10) for the storage of medical equipment, the kit comprising:
- a bag (12) made from a flexible plastics material;
- a first closure device (18) for securing the bag (12) closed, the closure device (18) being single use; and
- a second closure device (20) for securing the bag (12) closed, the closure device (20) being single use,
**characterized in that** the first closure device (18) has a distinguishing means (36) and an identification means, the second closure device (20) has a distinguishing means (36) and an identification means, the distinguishing means (36) of the first and second closure devices (18, 20) are different.

9. A kit (10) as claimed in Claim 8, wherein the identification means of the first and second closure devices (18, 20) are interrelated.

10. A kit as claimed in Claim 8 or Claim 9, wherein the first and second closure devices (18, 20) are cable ties.

11. A kit as claimed in Claim 10, wherein the cable ties (18, 20) each include a tab portion (30), the tab portion (30) being arranged such that, in use, a force applied to the tab portion (30) breaks the cable tie.

12. A kit as claimed in any of Claims 8 to 11, wherein the bag (12) has a rectangular base (14).

13. A kit as claimed in any of Claims 8 to 12, wherein the distinguishing means (36) comprises lettering on a part of each of the first and second closure devices (18, 20).

14. A kit as claimed in any of Claims 8 to 13, wherein the kit (10) further comprises a sheet of absorbent material.

15. An assembly comprising:
- a bag (12) made from a flexible plastics material;
- first and second closure devices (18, 20) for securing the bag (12) closed, the closure devices (18, 20) each being single use; and
- a piece of medical equipment contained within the bag (12),
wherein, one of the first and second closure devices (18, 20) is secured around the bag (12) to seal the bag closed
**characterized in that** the closure devices (18, 20) each have a distinguishing means (36) and an identification means, the distinguishing means (36) of the first and second closure devices (18, 20) being different.

16. An assembly as claimed in Claim 15, wherein the piece of medical equipment comprises an endoscope (16).

17. An assembly as claimed in Claim 15 or Claim 16, wherein the identification means of the first and second closure devices (18, 20) are interrelated.

18. An assembly as claimed in any of Claims 15 to 17, wherein the first and second closure devices (18, 20) are cable ties.

19. An assembly as claimed Claim 18, wherein the cable ties (18, 20) each include a tab portion (30), the tab portion (30) being arranged such that, in use, a force applied to the tab portion (30) breaks the cable tie.

20. An assembly as claimed in any of Claims 15 to 19, wherein the bag (12) has a rectangular base (14).

21. An assembly as claimed in any of Claims 15 to 20, wherein the distinguishing means (36) comprises lettering on a part of each of the first and second closure devices (18, 20).

22. An assembly as claimed in any of Claims 15 to 21, wherein the assembly further comprises a sheet of absorbent material positioned in the bag (12) for absorbing moisture from the piece of medical equipment.

## Patentansprüche

1. Verfahren zum Aufbewahren von medizinischer Ausrüstung, bei welchem ein Kit (10) zum Einsatz kommt, welches einen Beutel (12) aus einem flexiblen Kunststoffmaterial, eine erste Verschlusseinrichtung (18), um den Beutel geschlossen zu halten, und eine zweite Verschlusseinrichtung (20) umfasst, um den Beutel geschlossen zu halten, wobei das Verfahren Folgendes umfasst:
a) Platzieren eines medizinischen Ausrüstungsgegenstandes in einem ersten, sauberen Zustand innerhalb des Beutels (12);
b) Verschließen des Beutels (12) mit der ersten Verschlusseinrichtung (18);
c) Entfernen der ersten Verschlusseinrichtung (18);
d) Entfernen des medizinischen Ausrüstungsgegenstandes aus dem Beutel (12) zum Gebrauch;
e) Platzieren des gebrauchten medizinischen Ausrüstungsgegenstandes in einem zweiten, verunreinigten Zustand in dem Beutel (12); und
f) Verschließen des Beutels (12) mit der zweiten Verschlusseinrichtung (20) derart, dass der Beutel abgedichtet ist, um Feuchtigkeit innerhalb des Beutels (12) zu halten,
**dadurch gekennzeichnet,**
**dass** die erste Verschlusseinrichtung (18) ein Unterscheidungsmittel (36) und ein Identifikationsmittel umfasst, und die zweite Verschlusseinrichtung (20) ein Unterscheidungsmittel (36) und ein Identifikationsmittel umfasst, wobei die Unterscheidungsmittel (36) der ersten und zweiten Verschlusseinrichtung (18, 20) verschieden sind.

2. Verfahren nach Anspruch 1,
wobei die erste und die zweite Verschlusseinrichtung (18, 20) für den einmaligen Gebrauch ausgelegt sind und der Schritt des Entfernens der ersten Verschlusseinrichtung (18) das Zerstören der ersten Verschlusseinrichtung (18) beinhaltet.

3. Verfahren nach Anspruch 1 oder Anspruch 2,
wobei das Verfahren weiter das in Verbindung Bringen des Identifikationsmittels der ersten und zweiten Verschlusseinrichtung (18, 20) mit dem medizinischen Ausrüstungsgegenstand umfasst, um die Identifikation des medizinischen Ausrüstungsgegenstandes zu ermöglichen, wenn dieser sich innerhalb des versiegelten Beutels (12) befindet.

4. Verfahren nach einem der vorigen Ansprüche,
wobei das Kit (10) weiter einen Bogen aus Absorptionsmaterial umfasst, und wobei das Verfahren das Platzieren des Bogens aus Absorptionsmaterial in dem Beutel (12) umfasst, bevor der gebrauchte medizinische Ausrüstungsgegenstand in dem Beutel (12) platziert wird.

5. Verfahren nach einem der vorigen Ansprüche,
wobei die Verschlusseinrichtungen (18, 20) Kabelbinder sind.

6. Verfahren nach Anspruch 5,
wobei die Kabelbinder (18, 20) jeweils einen Laschenabschnitt (30) umfassen, wobei der Schritt des Entfernens der ersten Verschlusseinrichtung (18) das Ziehen des Laschenabschnittes (30) zum Zerstören des Kabelbinders (18) umfasst.

7. Verfahren nach einem der vorigen Ansprüche,
bei welchem der medizinische Ausrüstungsgegenstand ein Endoskop (16) ist.

8. Kit (10) für das Aufbewahren medizinischer Ausrüstungsgegenstände,
wobei das Kit Folgendes umfasst:
- einen Beutel (12) aus einem flexiblen Kunststoffmaterial;
- eine erste Verschlusseinrichtung (18), um den Beutel (12) geschlossen zu halten, wobei die Verschlusseinrichtung (18) zum einmaligen Gebrauch ausgebildet ist; und
- eine zweite Verschlusseinrichtung (20), um den Beutel (12) geschlossen zu halten, wobei die Verschlusseinrichtung (20) zum einmaligen Gebrauch ausgebildet ist,
**dadurch gekennzeichnet,**
**dass** die erste Verschlusseinrichtung (18) ein Unterscheidungsmittel (36) und ein Identifikationsmittel aufweist, und dass die zweite Verschlusseinrichtung (20) ein Unterscheidungsmittel (36) und ein Identifikationsmittel aufweist, wobei die Unterscheidungsmittel (36) der ersten und der zweiten Verschlusseinrichtung (18, 20) verschieden sind.

9. Kit (10) nach Anspruch 8,
wobei das Identifikationsmittel der ersten und zweiten Verschlusseinrichtung (18, 20) miteinander zusammenhängen.

10. Kit nach Anspruch 8 oder Anspruch 9,
wobei die erste und die zweite Verschlusseinrichtung (18, 20) Kabelbinder sind.

11. Kit nach Anspruch 10,
wobei die Kabelbinder (18, 20) jeweils einen Laschenabschnitt (30) enthalten, wobei der Laschenabschnitt (30) derart angeordnet ist, dass im Gebrauchszustand eine auf den Laschenabschnitt (30) ausgeübte Kraft den Kabelbinder zerstört.

12. Kit nach einem der Ansprüche 8 - 11,
wobei der Beutel (12) einen rechteckigen Grundabschnitt (14) aufweist.

13. Kit nach einem der Ansprüche 8 - 12,
bei welchem das Unterscheidungsmittel (36) eine Beschriftung auf einem Teil von jeweils der ersten und der zweiten Verschlusseinrichtung (18, 20) aufweist.

14. Kit nach einem der Ansprüche 8 - 13,
wobei das Kit (10) weiter einen Bogen aus Absorptionsmaterial umfasst.

15. Anordnung, umfassend:
- einen Beutel (12) aus einem flexiblen Kunststoffmaterial;
- erste und zweite Verschlusseinrichtungen (18, 20), um den Beutel (12) geschlossen zu halten, wobei die Verschlusseinrichtungen (18, 20) jeweils für den Einmalgebrauch ausgebildet sind; und
- ein Teil einer medizinischen Ausrüstung, der in dem Beutel (12) enthalten ist,
wobei die erste oder zweite Verschlusseinrichtung (18, 20) um den Beutel (12) herum gesichert ist, um den geschlossenen Beutel zu versiegeln,
**dadurch gekennzeichnet,**
**dass** die Verschlusseinrichtungen (18, 20) jeweils ein Unterscheidungsmittel (36) und ein Identifikationsmittel aufweisen, wobei das Unterscheidungsmittel (36) der ersten und zweiten Verschlusseinrichtung (18, 20) sich unterscheiden.

16. Anordnung nach Anspruch 15,
wobei das Teil einer medizinischen Ausrüstung ein Endoskop (16) umfasst.

17. Anordnung nach Anspruch 15 oder Anspruch 16,
wobei das Identifikationsmittel der ersten und zweiten Verschlusseinrichtungen (18, 20) miteinander zusammenhängen.

18. Anordnung nach einem der Ansprüche 15 - 17,
wobei die erste und zweite Verschlusseinrichtung (18, 20) Kabelbinder sind.

19. Anordnung nach Anspruch 18,
wobei die Kabelbinder (18, 20) jeweils einen Laschenabschnitt (30) enthalten, wobei der Laschenabschnitt (30) derart angeordnet ist, dass im Gebrauchszustand eine auf den Laschenabschnitt (30) ausgeübte Kraft den Kabelbinder zerstört.

20. Anordnung nach einem der Ansprüche 15 - 19,
wobei der Beutel (12) einen rechteckigen Grundabschnitt (14) aufweist.

21. Anordnung nach einem der Ansprüche 15 - 20,
bei welchem das Unterscheidungsmittel (36) eine Beschriftung auf einem Teil von jeweils der ersten und der zweiten Verschlusseinrichtung (18, 20) aufweist.

22. Anordnung nach einem der Ansprüche 15 - 21,
wobei die Anordnung weiter einen Bogen aus Absorptionsmaterial umfasst, welcher in dem Beutel (12) positioniert ist, um Feuchtigkeit aus dem Teil der medizinischen Ausrüstung zu absorbieren.

## Revendications

1. Procédé pour le rangement d'un équipement médical à l'aide d'un kit (10) comprenant un sac (12) fait d'une matière plastique souple, un premier dispositif de fermeture (18) pour fermer le sac et un second dispositif de fermeture (20) pour fermer le sac, le procédé comprenant
a) mettre en place un élément d'un équipement médical dans un premier état, propre, dans le sac (12) ;
b) fermer le sac (12) par le premier dispositif de fermeture (18) ;
c) enlever le premier dispositif de fermeture (18) ;
d) enlever l'équipement médical du sac (12) en vue de son utilisation ;
e) mettre en place ledit équipement médical utilisé, dans un second état, souillé, dans le sac (12) ; et
f) fermer le sac (12) par le second dispositif de fermeture (20), de telle sorte que le sac est scellé en vue de retenir l'humidité dans le sac (12),
**caractérisé par le fait que** le premier dispositif de fermeture (18) comprend un moyen de distinction (36) et un moyen d'identification, le second dispositif de fermeture (20) comprend un moyen de distinction (36) et un moyen d'identification, les moyens d'identification (36) des premier et second dispositifs de fermeture (18, 20) étant différents.

2. Procédé selon la revendication 1, dans lequel les premier et second dispositifs de fermeture (18, 20) sont à usage unique et l'étape d'enlèvement du premier dispositif de fermeture (18) comprend la rupture du premier dispositif de fermeture (18).

3. Procédé selon la revendication 1 ou la revendication 2, dans lequel le procédé comprend en outre l'association des moyens d'identification des premier et second dispositifs de fermeture (18, 20) avec l'élément d'équipement médical pour permettre l'identification dudit équipement médical lorsqu'il est scellé dans le sac (12).

4. Procédé selon l'une quelconque des revendications précédentes, dans lequel le kit (10) comprend en outre une feuille de matériau absorbant, et dans lequel le procédé comprend la mise en place de la feuille de matériau absorbant dans le sac (12) avant de placer l'élément d'équipement médical utilisé dans le sac (12).

5. Procédé selon l'une quelconque des revendications précédentes, dans lequel les dispositifs de fermeture (18, 20) sont des attaches de câble.

6. Procédé selon la revendication 5, dans lequel les attaches de câble (18, 20) comprennent chacune une partie languette (30), et dans lequel l'étape d'enlèvement du premier dispositif de fermeture (18) comprend la traction de la partie languette (30) de façon à rompre l'attache de câble (18).

7. Procédé selon l'une quelconque des revendications précédentes, dans lequel l'élément d'équipement médical est un endoscope (16).

8. Kit (10) pour le rangement d'un équipement médical, le kit comprenant :
- un sac (12) fait d'une matière plastique souple ;
- un premier dispositif de fermeture (18) pour fermer le sac (12), le dispositif de fermeture (18) étant à usage unique ; et
- un second dispositif de fermeture (20) pour fermer le sac (12), le dispositif de fermeture (20) étant à usage unique,
**caractérisé par le fait que** le premier dispositif de fermeture (18) a un moyen de distinction (36) et un moyen d'identification, le second dispositif de fermeture (20) a un moyen de distinction (36) et un moyen d'identification, les moyens d'identification (36) des premier et second dispositifs de fermeture (18, 20) étant différents.

9. Kit (10) selon la revendication 8, dans lequel les moyens d'identification des premier et second dispositifs de fermeture (18, 20) sont interdépendants.

10. Kit selon la revendication 8 ou la revendication 9, dans lequel les premier et second dispositifs de fermeture (18, 20) sont des attaches de câble.

11. Kit selon la revendication 10, dans lequel les attaches de câble (18, 20) comprennent chacune une partie languette (30), la partie languette (30) étant disposée de telle sorte que, lors de l'utilisation, une force appliquée à la partie languette (30) rompt l'attache de câble.

12. Kit selon l'une quelconque des revendications 8 à 11, dans lequel le sac (12) a une base rectangulaire (14).

13. Kit selon l'une quelconque des revendications 8 à 12, dans lequel le moyen de distinction (36) comprend un lettrage sur une partie de chacun des premier et second dispositifs de fermeture (18, 20).

14. Kit selon l'une quelconque des revendications 8 à 13, dans lequel le kit (10) comprend en outre une feuille de matériau absorbant.

15. Ensemble comprenant :
- un sac (12) fait d'une matière plastique souple;
- des premier et second dispositifs de fermeture (18, 20) pour fermer le sac (12), les dispositifs de fermeture (18, 20) étant chacun à usage unique ; et
- un élément d' un équipement médical contenu dans le sac (12),
l'un des premier et second dispositifs de fermeture (18, 20) étant fixé autour du sac (12) pour fermer le sac de façon scellée,
**caractérisé par le fait que** les dispositifs de fermeture (18, 20) ont chacun un moyen de distinction (36) et un moyen d'identification, les moyens de distinction (36) des premier et second dispositifs de fermeture (18, 20) étant différents.

16. Ensemble selon la revendication 15, dans lequel l'élément d'équipement médical comprend un endoscope (16).

17. Ensemble selon la revendication 15 ou la revendication 16, dans lequel les moyens d'identification des premier et second dispositifs de fermeture (18, 20) sont interdépendants.

18. Ensemble selon l'une quelconque des revendications 15 à 17, dans lequel les premier et second dispositifs de fermeture (18, 20) sont des attaches de câble.

19. Ensemble selon la revendication 18, dans lequel les attaches de câble (18, 20) comprennent chacune une partie languette (30), la partie languette (30) étant disposée de telle sorte que, lors de l'utilisation, une force appliquée à la partie languette (30) rompt l'attache de câble.

20. Ensemble selon l'une quelconque des revendications 15 à 19, dans lequel le sac (12) a une base rectangulaire (14).

21. Ensemble selon l'une quelconque des revendications 15 à 20, dans lequel le moyen de distinction (36) comprend un lettrage sur une partie de chacun des premier et second dispositifs de fermeture (18, 20).

22. Ensemble selon l'une quelconque des revendications 15 à 21, dans lequel l'ensemble comprend en outre une feuille de matériau absorbant positionnée dans le sac (12) pour absorber l'humidité provenant de l'élément d'équipement médical.
